(19) **Europäisches Patentamt / European Patent Office / Office européen des brevets**

(11) **EP 1 874 329 B1**

(12) **EUROPEAN PATENT SPECIFICATION**

(45) Date of publication and mention
of the grant of the patent:
**15.07.2009 Bulletin 2009/29**

(21) Application number: **06754893.3**

(22) Date of filing: **27.04.2006**

(51) Int Cl.:
$C07K\ 5/08^{(2006.01)}$      $A61K\ 38/06^{(2006.01)}$
$C12P\ 21/02^{(2006.01)}$      $C12P\ 21/06^{(2006.01)}$
$A23L\ 1/305^{(2006.01)}$      $A61P\ 9/12^{(2006.01)}$

(86) International application number:
**PCT/EP2006/061883**

(87) International publication number:
**WO 2006/114441 (02.11.2006 Gazette 2006/44)**

(54) **BLOOD PRESSURE LOWERING PROTEIN HYDROLYSATES**

BLUTDRUCKSENKENDE PROTEINHYDROLYSATE

HYDROLYSATS PROTEIQUES ABAISSANT LA PRESSION ARTERIELLE

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **28.04.2005 EP 05103525**

(43) Date of publication of application:
**09.01.2008 Bulletin 2008/02**

(73) Proprietor: **DSM IP Assets B.V.
6411 TE Heerlen (NL)**

(72) Inventors:
• **EDENS, Luppo
NL-3062 JL Rotterdam (NL)**

• **DE ROOS, Andre Leonardus
NL-2614 TB Delft (NL)**
• **VAN PLATERINK, Christianus, Jacobus
NL-3132 RP Vlaardingen (NL)**

(74) Representative: **Matulewicz, Emil Rudolf Antonius
et al
DSM Intellectual Property
Delft Office - PP 600-0240
P.O. Box 1
2600 MA Delft (NL)**

(56) References cited:
**EP-A- 0 583 074**      **WO-A-01/68114**
**WO-A-01/81366**      **WO-A-01/81368**

**Description**

Field of the invention

The present invention relates to the production of novel peptides.

Background of the invention

Hypertension is a relatively common disease state in humans and presents a prevalent risk factor for cardiovascular diseases, kidney failure and stroke. The availability of a large array of pharmaceutical products such as calcium blockers, beta blockers, diuretics, alpha blockers, central alpha antagonists, angiotensin II antagonists and ACE inhibitors, illustrates that the underlying physiological mechanisms for hypertension are manysided.

Of the physiological mechanisms for hypertension, especially the renin-angiotensin mechanism has received a lot of scientific attention. In this mechanism, angiotensin is secreted by the liver and is cleaved by the peptidase renin to yield the biologically inactive decapeptide angiotensin I. As angiotensin I passes through the lung capillaries, another peptidase called angiotensin converting enzyme (hereinafter referred to as ACE) acts on angiotensin I by removing the last two residues of angiotensin I (His-Leu) to form the octapeptide angiotensin II. The angiotensin II octapeptide exhibits strong vasoconstricting activity and therefore raises blood pressure. ACE inhibition leading to lower levels of the angiotensin II prevents vasoconstriction and thus high blood pressures.

Apart from cleaving angiotensin I, ACE can also hydrolyse bradykinin, a nonapeptide also participating in blood pressure regulation. In the latter mechanism ACE inhibition leads to increased bradykinine levels which promote vasodilatation and lower blood pressure as well. Inhibiting ACE thus leads to blood pressure lowering effects via at least two separate mechanisms.

It is also known that the octapeptide angiotensin II stimulates the release of aldosterone by the adrenal cortex. The target organ for aldosterone is the kidney where aldosterone promotes increased reabsorbtion of sodium from the kidney tubules. Also via this third mechanism ACE inhibition reduces blood pressure but in this case by diminishing sodium reabsorption.

Because of its multiple physiological effects, inhibiting the proteolytic activity of ACE is an effective way of depressing blood pressure. This observation has resulted in a number of effective pharmaceutical blood pressure lowering products such as captopril and enalapril (Ondetti, M.A. et al., 1977, Science, Washington DC, 196, 441-444).

Because hypertension is a relatively common disease state it would be advantageous to counteract this undesirable result of modern life style with mildly active natural ingredients. Especially mildly active natural ingredients that can be incorporated into food or beverage products because such products are consumed on a regular basis. Alternatively such mildly active natural ingredients could be incorporated into dietary supplements. During the last decades it has been discovered that specific peptides present in fermented milk have an ACE inhibiting capacity and can induce blood pressure reductions in hypertensive subjects. Nowadays numerous in vitro and a few animal trials have demonstrated ACE inhibiting effects of different peptides obtained from a variety of protein sources. Although in vitro ACE inhibition assays have revealed many different peptide sequences, it has to be emphasized that ACE inhibiting peptides need to circulate in the blood to exert an in vivo effect. The implication is that efficacious ACE inhibiting peptides should resist degradation by the gastrointestinal proteolytic digestion system and should remain intact during a subsequent transport over the intestinal wall.

A structure-function study of the various ACE inhibiting peptides has suggested that they often possess a Pro-Pro, Ala-Pro or Ala-Hyp at their C-terminal sequence (Maruyama, S. and Suzuki, H., 1982; Agric Biol Chem., 46(5): 1393-1394). This finding is partly explained by the fact that ACE is a peptidyl dipeptidase (EC3.4.15.1) unable to cleave peptide bonds involving proline. Thus from tripeptides having the structure Xaa-Pro-Pro the dipeptide Pro-Pro cannot be removed because the Xaa-Pro bond cannot be cleaved. It is therefore conceivable that if present in relatively high concentrations, tripeptides having the Xaa-Pro-Pro structure will inhibit ACE activity. As not only ACE but almost all proteolytic enzymes have difficulties in cleaving Xaa-Pro or Pro-Pro bonds, the notion that the presence of (multiple) proline residues at the carboxyterminal end of peptides results in relatively protease resistant molecules is almost self-evident. Similarly peptides containing hydroxyproline (Hyp) instead of proline are relatively protease resistant. From this it can be inferred that peptides carrying one or more (hydroxy)proline residues at their carboxyterminal end are likely to escape from proteolytic degradation in the gastro-intestinal tract. These conclusions will help us to understand the remarkable in vivo blood pressure lowering effect of specific ACE inhibiting peptides: not only do they meet the structural requirements for ACE inhibition, they also resist degradation by the gastrointestinal proteolytic digestion system and remain intact during a subsequent transport over the intestinal wall.

Strong ACE inhibiting activities have been reported for the tripeptides Leu-Pro-Pro (JP02036127), Val-Pro-Pro (EP 0 583 074) and Ile-Pro-Pro (J. Dairy Sci., 78:777-7831995)). Initially all ACE inhibiting peptides were characterized on the basis of their in vitro effect on ACE activity and the tripeptides Ile-Pro-Pro (hereinafter referred to as IPP) Val-Pro-Pro (hereinafter referred to as VPP) and Leu-Pro-Pro (hereinafter referred to as LPP) stood out because of their strong ACE inhibiting effect resulting in relatively low IC50 values. Later on the presumed antihypertensive effects of the tripeptides VPP as well as IPP could be confirmed in spontaneously hypertensive rats (Nakamura et al., J. Dairy Sci., 78:12531257 (1995)). In these experiments the inhibitory tripeptides were derived from lactic acid bacteria fermented milk. During the milk fermentation the desirable peptides are produced by proteinases produced by the growing lactic acid bacteria. ACE inhibiting peptides have been concentrated from fermented milk products after electrodialysis, hollow fiber membrane dialysis or chromatographic methods to enable their marketing in the form of concentrated dietary supplements like tablets or lozenges.

Summary of the invention

The present invention relates to the novel tripeptides MAP and/or ITP or to a salt of MAP and/or a salt of ITP.
The present invention also relates to a protein hydrolysate comprising MAP and/or ITP or a salt of MAP and/or ITP. Preferably the protein hydrolysate has a degree of hydrolysis (DH) of 5 to 50%, more preferably 10 to 40% and most preferably a DH of 2d to 35%.
Furthermore the present invention relates to a peptide mixture comprising MAP and/or ITP or a salt of MAP and/or ITP. Preferably this peptide mixture comprises at least 1 mg MAP/g protein, more preferably at least 2 mg/g and most preferably at least 4 mg/g protein. Preferably the peptide mixture also comprises LPP and/or IPP. Therefore the peptide mixture preferably also comprises at least 1 mg IPP/g protein, more preferably at least 2 mg/g and most preferably at least 4 mg/g protein and/or the peptide mixture preferably also comprises at least 1 mg LPP/g protein, more preferably at least 2 mg/g and most preferably at least 4 mg/g protein.
These peptide mixtures preferably comprise at least 30 wt% (dry matter) peptides having a MW of less than 500Da, more preferably 35 to 70% wt (dry matter) of the peptide mixture are peptides having a MW of less than 500Da.
Furthermore the present invention relates to an enzymatic process to produce MAP and/or ITP, preferably by enzymatic hydrolysis of a protein source.

Detailed description of the invention

As shown in the present application the tripeptides MAP and ITP are quite effective in inhibiting ACE. These inhibition effects correspond to surprisingly low IC50 values, respectively 0.4 for MAP and 10 for ITP (in $\mu$M) as determined in the experimental part herein. Moreover, it is found that both tripeptides MAP and ITP resist gastrointestinal proteolytic degradation and are thus expected to be stable in the human intestinal tract. The tripeptide MAP and/or the tripeptide ITP and salts thereof are therefore very suitable for an effective ACE inhibition and can for example be used in functional food, as a nutraceutical or as a medicament. The term nutraceutical as used herein denotes the usefulness in both the nutritional and pharmaceutical field of application. Thus, novel nutraceutical compositions comprising MAP and/or ITP can find use as supplement to food and beverages and as pharmaceutical formulations or medicaments for enteral or parenteral application which may be solid formulations such as capsules or tablets, or liquid formulations, such as solutions, suspensions or emulsions.
The tripeptides MAP (Met-Ala-Pro) and ITP (Ile-Thr-Pro) can be made by a variety of methods including chemical synthesis, enzymatic hydrolysis and fermentation of protein containing solutions.
The identification of biologically active peptides in complex mixtures such as protein hydrolysates or liquids resulting from fermentation is a challenging task. Apart from the basic questions: are we using the right protein substrate, are we using the right enzyme, are we using the right microbial culture, several biologically active peptides can be expected to be present in complex samples containing thousands of peptides. The traditional identification approaches employing repeated cycles of high-performance liquid chromatographic (HPLC) fractionation and biochemical evaluation are generally time consuming and prone to losses of the biologically active peptides present making the detection of relevant bio-activity extremely difficult. In the present work very sophisticated equipment was used and many different protein hydrolysates and fermentation broths were screened finally leading us to the identification of the two novel peptides MAP and ITP which have ACE inhibitory properties. In our approach a continuous flow biochemical assay was coupled on-line to an HPLC fractionation system. The HPLC column effluent was split between a continuous flow ACE bioassay and a chemical analysis technique (mass spectrometry). Crude hydrolysates and fermentation broths were separated by HPLC, after which the presence of biologically active compounds was detected by means of the on-line biochemical assay. Mass spectra were recorded continuously so that structural information was immediately available when a peptide shows a positive signal on the biochemical assay.
The tripeptides MAP and ITP as identified by the above mentioned approach can be produced by various methods including economically viable production routes. Production via chemical synthesis is possible using conventional techniques as for instance described in "Peptides: Chemistry and Biology" by N. Sewald and H.D. Jakubke, Eds. Wiley-VCH Verlag GmbH, 2002, Chapter 4. Particular cost-effective methods of chemical peptide synthesis suitable for large-scale production are based on the use of alkylchloroformates or pivaloyl chloride for the activation of the carboxylic group combined with the use of methyl esters for C-terminal protection and benzyloxycarbonyl (Z) or tert-butyloxycarbonyl groups for N-protection. For instance, in the case of MAP, L-proline methylester can be coupled with isobutylchloroformate-activated Z-Ala; the resulting dipeptide can be Z-deprotected through hydrogenolysis using hydrogen and Pd on C and coupled again with isobutylchloroformate-activated Z-Met; of the resulting tripeptide the methyl ester is hydrolyzed using NaOH and after Z-deprotection by hydrogenolysis the tripeptide Met-Ala-Pro is obtained. Similarly, Ile-Thr-Pro can be synthesized but during the coupling reactions the hydroxy function of Thr requires benzyl-protection; in the final step this group is then simultaneously removed during the Z-deprotection.

MAP and/or ITP may also be made by enzymatic hydrolysis or by fermentative approaches using any protein substrate containing the amino acid sequences MAP and/or ITP. Advantageously the protein substrate contains both fragments MAP and ITP. Prefered protein substrates for such enzymatic or fermentative approaches are bovine milk or the casein fraction of bovine milk. Through optimisation of the fermentation or hydrolysis conditions, the production of the biologically active molecules MAP and/or ITP may be maximised. The skilled person trying to maximise the production will know how to adjust the process parameters, such as hydrolysis/fermentation time, hydrolysis/fermentation temperature, enzyme/microorganism type and concentration etc.

MAP and/or ITP or compositions comprising MAP and/or ITP are advantageously hydrolysates and preferably made according to a process involving the following steps:

(a) enzymatic hydrolysis of a suitable protein substrate comprising MAP or ITP in its amino acid sequence resulting in a hydrolysed protein product comprising the tripeptides MAP and/or ITP;
(b) separation from the hydrolysed protein product of a fraction rich in tripeptide MAP and/or the tripeptide ITP; and optionally
(c) concentrating and/or drying the fraction from step b) to obtain a concentrated liquid or a solid rich in tripeptide MAP and/or the tripeptide ITP.

The enzymatic hydrolysis step (a) may be any enzymatic treatment of the suitable protein substrate leading to hydrolysis of the protein resulting in liberation of MAP and/or ITP tripeptides. Although several enzyme combinations can be used to release the desired tripeptides from the protein substrate, the preferred enzyme used in the present process is a proline specific endoprotease or a proline specific oligopeptidase. A suitable protein substrate may be any substrate encompassing the amino acid sequence MAP and/or ITP. Protein substrates known to encompass MAP are, for example, casein, wheat gluten, sunflower protein isolate, rice protein, egg protein. Suitable protein substrates preferably encompass the amino acid sequences AMAP or PMAP as occur in beta-casein bovine, the alpha-gliadin fraction of wheat gluten and in the 2S fraction of sunflower protein isolate.

The casein substrate may be any material that contains a substantial amount of beta-casein and alpha-s2-casein. Examples of suitable substrates are milk as well as casein, casein powder, casein powder concentrates, casein powder isolates, or beta-casein; or alpha-s2-casein. Preferably a substrate that has a high content of casein, such as casein protein isolate (CPI).

The enzyme may be any enzyme or enzyme combination that is able to hydrolyse protein such as beta-casein and/or alpha-s2-casein resulting in the liberation of one or more of the tripeptides of MAP and/or ITP.

The separation step (b) may be executed in any way known to the skilled person, e.g. by precipitation, filtration, centrifugation, extraction or chromatography and combinations thereof. Preferably the separation step (b) is executed using micro- or ultrafiltration techniques. The pore size of the membranes used in the filtration step, as well as the charge of the membrane may be used to control the separation of the tripeptide MAP and/or the tripeptide ITP. The fractionation of casein protein hydrolysates using charged UF/NF membranes is described in Y. Poilot et al, Journal of Membrane Science 158 (1999) 105-114.

The concentration step (c) may involve nanofiltration or evaporation of the fraction generated by step (b) to yield a highly concentrated liquid. If suitably formulated, e.g. with a low water activity (Aw), a low pH and preferably a preservative such as benzoate or sorbate, such concentrated liquid compositions form an attractive way of storage of the tripeptides according to the invention. Optionally the evaporation step is followed by a drying step e.g. by spray drying or freeze drying to yield a solid containing a high concentration of MAP and/or ITP.

The enzymatic process comprises preferably a single enzyme incubation step.
The enzymatic process according to the present invention further relates to the use of a proline specific protease which is preferably free of contaminating enzymatic activities. A proline specific protease is defined as a protease that hydrolyses a peptide bond at the carboxy-terminal side of proline. The preferred proline specific protease is an protease that hydrolyses the peptide bond at the carboxy terminal side of proline and alanine residues. The proline specific protease is preferably capable of hydrolyzing large protein molecules like polypeptides or the protein itself. The process according to the invention has in general an incubation time of less than 24 hours, preferably the incubation time is less than 10 hours and more preferably less than 4 hours. The incubation temperature is in general higher than 30°C, preferably higher than 40°C and more preferably higher than 50°C.

Another aspect of the present invention is the purification and/or separation of the tripeptides MAP and ITP from a hydrolysed protein. Most of the hydrolysed protein according to the invention is preferably capable to precipitate under selected pH conditions. This purification process comprises altering the pH to the pH whereby most of the hydrolysed and unhydrolysed protein precipitates and separating the precipitated proteins from the (bio-active) tripeptides that remain in solution.

To obtain the present tripeptides with a proline residue at their carboxyterminal end, the use of a protease that can cleave at the carboxyterminal side of proline residues offers a preferred option. Socalled prolyl oligopeptidases (EC 3.4.21.26) have the unique possibility of preferentially cleaving peptides at the carboxyl side of proline residues. Prolyl oligopeptidases also have the possibility to cleave peptides at the carboxyl side of alanine residues, but the latter reaction is less efficient than cleaving peptide bonds involving proline residues. In all adequately characterized proline specific proteases isolated from mammalian as well as microbial sources, a unique peptidase domain has been identified that excludes large peptides from the enzyme's active site. In fact these enzymes are unable to degrade peptides containing more than about 30 amino acid residues so that these enzymes are now referred to as "prolyl oligopeptidases" (Fulop et al : Cell, Vol. 94, 161-170, July 24,1998). As a consequence these prolyl oligopeptidases require a pre-hydrolysis with other endoproteases before they can exert their hydrolytic action. However, as described in WO 02/45523, even the combination of a prolyl oligopeptidase with such another endoprotease results in hydrolysates characterized by a significantly enhanced proportion of peptides with a carboxyterminal proline residue. Because of this, such hydrolysates form an excellent starting point for the isolation of the tripeptides with in vitro ACE inhibiting effects as well as an improved resistance to gastro-intestinal proteolytic degradation.

A "peptide" or "oligopeptide" is defined herein as a chain of at least two amino acids that are linked through peptide bonds. The terms "peptide" and "oligopeptide" are considered synonymous (as is commonly recognized) and each term can be used interchangeably as the context requires. A "polypeptide" is defined herein as a chain containing more than 30 amino acid residues. All (oligo)peptide and polypeptide formulas or sequences herein are written from left to right in the direction from amino-terminus to carboxy-terminus, in accordance with common practice. The one-letter code of amino acids used herein is commonly known in the art and can be found in Sambrook, et al. (Molecular Cloning: A Laboratory Manual, 2nd,ed. Cold Spring Harbor Laboratory, Cold Spring Harbor Laboratory Press, Cold Spring Harbor, NY, 1989). A peptide mixture is a composition comprising at least 30% wt (dry matter) of peptides determined on the based Kjeldahl Nitrogen in combination with determination of the peptide having a MW of lower than 3500Da.

An endoprotease is defined herein as an enzyme that hydrolyses peptide bonds in a polypeptide in an endo-fashion and belongs to the group EC 3.4. The endoproteases are divided into sub-subclasses on the basis of catalytic mechanism. There are sub-subclasses of serine endoproteases (EC 3.4.21), cysteine endoproteases (EC 3.4.22), aspartic endoproteases (EC 3.4.23), metalloendoproteases (EC 3.4.24) and threonine endoproteases (EC 3.4.25). Exoproteases are defined herein as enzymes that hydrolyze peptide bonds adjacent to a terminal $\alpha$-amino group ("aminopeptidases"), or a peptide bond between the terminal carboxyl group and the penultimate amino acid ("carboxypeptidases").

WO 02/45524 describes a proline specific protease obtainable from Aspergillus niger. The A. niger derived enzyme cleaves preferentially at the carboxyterminus of proline, but can also cleave at the carboxyterminus of hydroxyproline and, be it with a lower efficiency, at the carboxyterminus of alanine. WO 02/45524 also teaches that there exists no clear homology between this A. niger derived enzyme and the known prolyl oligopeptidases from other microbial or mammelian sources. In contrast with known prolyl oligopeptidases, the A.niger enzyme has an acidic pH optimum. Although the known prolyl oligopeptidases as well as the A. niger derived enzyme are socalled serine proteases, we show here (Example 1) that the A. niger enzyme belongs to a completely different subfamily. The secreted A. niger enzyme appears to be a member of family S28 of serine peptidases rather than the S9 family into which most cytosolic prolyl oligopeptidases have been grouped (Rawling,N.D. and Barrett, A.J.; Biochim. Biophys. Acta 1298 (1996) 1-3). In Example 2 we show the pH and temperature optima of the A. niger derived proline specific protease. In Example 3 we illustrate the high preference of the A. niger derived proline specific endoprotease for cleaving carboxyterminal of proline and alanine residues. Furthermore we demonstrate in this Example that the A. niger derived enzyme preparation as used in the process of the present invention is preferably essentially pure meaning that no significant endoproteolytic activity other than the endoproteolytic activity inherent to the pure proline specific endoprotease is present. We also demonstrate that our A. niger derived enzyme preparation preferably used according to the present invention does not contain any exoproteolytic, more specifically aminopeptidolytic side activities. Preferably exoproteolytic activity is absent in the A. niger derived enzyme preparation used in the process of the invention. Experimental proof for the notion that the proline specific endoproteolytic activity is essentially absent in non-recombinant Aspergillus strains can be found in WO 02/45524 and is also illustrated in Example 3 of the present application. Because the process of the present invention is possible by incubating the casein substrate with only the proline specific endoprotease, the optimal incubation conditions like temperature, pH etc. can be easily selected and does not have to be fixed at sub optimal conditions as would be the case if two or more enzymes are applied. Furthermore the formation of unwanted side products as for example additional, non-bio-active peptides or free amino acids leading to brothy off tastes is prevented. Having more degrees of freedom in selecting the reaction conditions makes an easier selection for other criteria possible. For example it is much easier to select now conditions which are less sensitive to microbial infections and to optimise pH conditions relative to subsequent protein precipitation steps. In Example 4 we show that the Aspergillus enzyme is not an oligopeptidase but a true endopeptidase able to hydrolyse intact proteins, large peptides as well as smaller peptide molecules without the need of an accessory endoprotease. This new and surprising finding opens up the possibility of using the A.niger enzyme for preparing hydrolysates with unprecedented high contents of peptides with a carboxyterminal proline residue because no accessory endoprotease is required. Such new hydrolysates can be prepared from different proteinaceous starting materials be it from vegetable or from animal origin. Examples of such starting materials are caseins, gelatin, fish or egg proteins, wheat gluten, soy and pea protein as well as rice protein and sunflower protein. As sodium is known to play an important role in hypertension, preferred substrates for the production of ACE inhibiting peptides are calcium and potassium rather than sodium salts of these proteins.

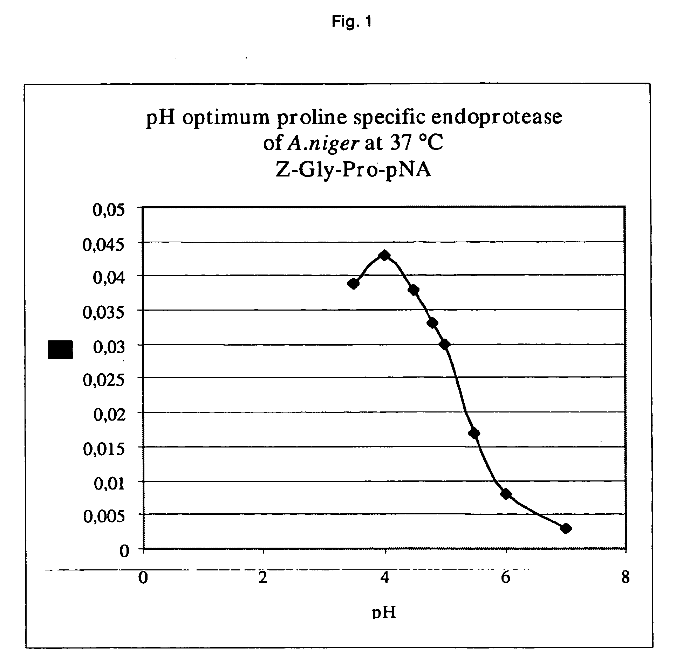

The pH optimum of the A. niger derived prolyl endoprotease is around 4.3. (see figure 1). Because of this low pH optimum incubating bovine milk caseinate with the A. niger derived prolyl endoprotease is not self-evident. Bovine milk caseinate will precipitate if the pH drops below 6.0 but at pH 6.0 the A. niger enzyme has a limited activity only. However, we show in Example 5 that even under this rather unfavorable condition an incubation with the A.niger derived prolyl endoprotease can yield several known ACE inhibiting peptides such as IPP and LPP. Quite surprisingly no VPP is produced under these conditions. Bovine milk casein incorporates a number of different proteins including beta-casein and kappa-casein. According to the known amino sequences beta-casein encompasses the ACE inhibitory tripeptides IPP, VPP and LPP. Kappa-casein encompasses IPP only. The fact that the A. niger derived enzyme does not contain any measurable aminopeptidase activity strongly suggests that the IPP formed is released from the -A107-I108-P109-P110- sequence present in kappa-caseine. Presumably the peptide bond carboxyterminal of IPP is cleaved by the main activity of the A. niger derived prolyl endoprotease whereas cleavage of the preceding Ala-Ile bond is accomplished by its Ala-specific side activity. Similarly the absence of VPP can be explained on the basis of the absence of aminopeptidase side activity. VPP is contained in beta-casein in the sequence $-P_{81}-V_{82}-V_{83}-V_{84}-P_{85}-P_{88}-$. So the proline specific endo-protease excises the VVVPP sequence but is unable to release VPP.

These results are obtained upon incubating the caseinate with the A. niger derived endoprotease in a simple one-step enzyme process. Aqueous solutions containing protein are highly susceptible for microbial infections, especially if kept for many hours at pH values above 5.0 and at temperatures of 50 degrees C or below. Especially microbial toxins that can be produced during such prolonged incubation steps and are likely to survive subsequent heating steps and form a potential threat to food grade processes. The present invention preferably uses an incubation temperature above 50 degrees C. In combination with the one-step enzyme process in which the enzyme incubation is carried out for a period less than 24 hours, preferably less than 8 hours, more preferably less than 4 hours, the process according to the invention offers the advantage of an improved microbiological stability. Using the present enzyme-substrate ratio in combination with the high temperature conditions, the excision of IPP and LPP is completed within a 3 hours incubation period.

Because the ACE inhibiting peptides IPP and LPP can be excised from casein using a single, essentially pure endoprotease, the present invention results in a smaller number of water soluble peptides than in the prior art processes. Among these water soluble peptides IPP an LPP are present in major amounts. This is especially important in case a high concentration of ACE inhibiting tripeptides is needed without many other, often less active compounds.

According to the present process preferably at least 20%, more preferably at least 30%, most preferably at least 40% of an -I-P-P- or an -L-P-P- sequence present in a protein is converted into the tripeptide IPP or LPP, respectively.

In Example 6 we illustrate the 5-fold purification effect of the bio-active peptides by a new and surprising purification step. The basis of this purification process is formed by the unique properties of the A. niger derived proline specific endoprotease. Incubation with this enzyme releases the most bio-active parts of the substrate molecule in the form of water-soluble tripeptides. The non- or less-bioactive parts of the substrate molecule remain to a large extent in non-cleaved and therefore much larger peptide or polypeptide parts of the substrate molecules. Due to the limited water solubilities of these larger peptide or polypeptide parts under selected pH conditions, these non- or less bioactive parts of the substrate molecule are easily separated from the much more soluble bio-active tripeptides. In this process the initial hydrolysate is formed during the brief enzyme incubation period at 55 degrees C, pH 6.0 and is then optionally heated to a temperature above 80 degrees C to kill all contaminating microorganisms and to inactivate the A. niger derived prolyl endopeptidase. Subsequently the hydrolysate is acidified to realise a pH drop to 4.5 or at least below 5.0. At this pH value, which cannot be used to inactivate the A. niger derived prolyl endopeptidase because it represents the optimum condition for the enzyme, all large peptides from the caseinate precipitate so that only the smaller peptides remain in solution. As the precipitated caseinates can be easily removed by decantation or a filtration step or a low speed (i.e. below 5000 rpm) centrifugation, the aqueous phase contains a high proportion of bioactive peptides relative to the amount of protein present. According to Kjeldahl data 80 to 70 % of the caseinate protein is removed by the low speed centrifugation step which implies a four- to five-fold purification of the ACE inhibiting peptides. We have found that this purification principle can be advantageously applied to obtain biologically active peptides obtained from proteinaceous material other than casein as well. Also not only enzymatically produced hydrolysates but also proteins that are fermentated by suitable microorganisms can be separated and purified according to the present process. Incubating enzyme and substrate at a pH value close to where the substrate will precipitate and where the enzyme is still active, will permit this purification step. Due to the low pH optimum of the A. niger derived prolyl endoprotease, substrate precipitations in the range between pH 1.5 to 6.5 can be considered. In view of their specific precipitation behaviour, gluten precipitations above pH 3.5, sun flower protein precipitations above pH 4.0 and below pH 6.0, egg white precipitations above pH 3.5 and below pH 5.0 form examples of conditions whereby the hydrolysed protein precipitates and the precipitated proteins can be separated from the hydrolysed protein or peptides.

After decantation, filtration or low speed centrifugation, the supernatants containing the biologically active peptides can be recovered in a purified state. A subsequent evaporation and spray drying step will yield an economical route for obtaining a food grade paste or powder with a high bio-activity. Upon the digestion of caseinates according to the process as described, a white and odourless powder with a high concentration of ACE inhibiting peptides, is obtained. Alternatively evaporation or nanofiltration can be used to further concentrate the bio-active peptides. The proper formulation of such a concentrate by increasing the water activity (Aw) in combination with a pH adjustment and the addition of a food grade preservative like a benzoate or a sorbate will yield a microbiologically stabilized, food grade, liquid concentrate of the blood pressure lowering peptides. If appropriately diluted to the right tripeptide concentration, a versatile starting material is obtained suitable for endowing all kinds of foods and beverages with ACE inhibiting properties. If required, the supernatant obtained after the decantation, filtration or low speed centrifugation can be further processed to improve the palatability of the final product. For example, the supernatant can be contacted with powdered activated charcharcoal followed by a filtration step to remove the charcoal. To minimise bitterness of the final product, the supernatant obtained after the decantation, filtration or low speed centrifugation can also be subjected to an incubation with another protease, such as subtilisin, trypsin, a neutral protease or a glutamate-specific endoprotease. If required, the concentration of the bioactive ingredients MAP and/or ITP can be increased even further by subsequent purification steps in which use is made of the specific hydrophilic/hydropholic character of the tripeptides MAP and ITP. Preferred purification methods include nanofiltration (separation on size), extraction for example with hexane or butanol followed by evaporation/precipitation or contacting the acidified hydrolysate as obtained with chromatographic resins from the Amberlite XAD range (Roehm). Also butyl-sepharose resins as supplied by Pharmacia can be used.

In Example 7 we describe the identification of the new ACE inhibiting peptides MAP and ITP in a casein hydrolysate prepared using the A. niger derived proline specific endoprotease in combination with the new peptide purification process. Only the use of this single and (essentially pure) endoprotease in combination with the removal of a large proportion of the non-bio-active peptides and highly sophisticated separation and identification equipment has allowed us to trace and identify these new ACE inhibiting tripeptides. In the casein derived bioactive peptides (CDBAP) prepared according to the Example 7 (after precipitation), the tripeptides MAP and ITP were identified in quantities corresponding with 2.9 mg MAP/gram CDBAP (4.8 mg MAP/gram protein in CDBAP) and 0.9 mg ITP/ gram CDBAP (1.4 mg ITP/ gram protein in CDBAP). A further characteristic for CDBAP is its extraordinary high proline content of 24% on molar basis. The tests described in this Example 7 illustrate the very low IC50 values for the two new tripeptides in the Modified Matsui test i.e. 0.5 micromol/l for MAP and 10 micromol/l for ITP. This finding is even more surprising if we realize that IPP, one of the most effective natural ACE inhibiting peptides known, has an IC50 value in this Modified Matsui test of 2.0 micromol/l.

According to the present process preferably at least 20%, more preferably at least 30%, most preferably at least 40% of an -M-A-P- or an -I-T-P- sequence present in a protein is converted into the tripeptide MAP or ITP, respectively.

The usefulness of the newly identified ACE inhibiting peptides MAP and ITP is further illustrated in Example 8. In the latter Example we show that both peptides survive incubation conditions simulating the digestive conditions typically found in the gastro-intestinal tract. On the basis of these data we conclude that the novel tripeptides are likely to survive in the mammalian (for example human) gastrointestinal tract implying a considerable economic potential if used to treat hypertension.

In Example 9 we demonstrate that the superior ACE inhibiting peptide MAP cannot only be produced in enzymatic hydrolysis experiments but is also detectable in milk preparations fermented with an appropriate food grade microorganism. However, we have been unable to demonstrate the presence of peptide ITP in such a fermented product.

The peptides MAP and/or ITP as obtained either before or after an additional (for example chromatographic purification steps may be used for the incorporation into food products that are widely consumed on a regular basis. Examples of such products are margarines, spreads, various dairy products such as butter or yoghurts or milk or whey containing beverages. Although such compositions are typically administered to human beings, they may also be administered to animals, preferably mammals, to relief hypertension. Furthermore the high concentration of ACE inhibitors in the products as obtained makes these products very useful for the incorporation into dietary supplements in the form off pills, tablets or highly concentrated solutions or pastes or powders. Slow release dietary supplements that will ensure a continuous release of the ACE inhibiting peptides are of particular interest. The MAP and/or ITP peptides according to the invention may be formulated as a dry powder in, for example, a pill, a tablet, a granule, a sachet or a capsule. Alternatively the enzymes according to the invention may be formulated as a liquid in, for example, a syrup or a capsule. The compositions used in the various formulations and containing the enzymes according to the invention may also incorporate at least one compound of the group consisting of a physiologically acceptable carrier, adjuvant, excipient, stabiliser, buffer and diluant which terms are used in their ordinary sense to indicate substances that assist in the packaging, delivery, absorption, stabilisation, or, in the case of an adjuvant, enhancing the physiological effect of the enzymes. The relevant background on the various compounds that can be used in combination with the enzymes according to the invention in a powdered form can be found in "Pharmaceutical Dosage Forms", second edition, Volumes 1,2 and 3, ISBN 0-8247-8044-2 Marcel Dekker, Inc. Although the ACE inhibiting peptides according to the invention formulated as a dry powder can be stored for rather long periods, contact with moisture or humid air should be avoided by choosing suitable packaging such as for example an aluminium blister. A relatively new oral application form is the use of various types of gelatin capsules or gelatin based tablets.

In view of the relevance of natural ACE inhibiting peptides to fight hypertension the present new and cost effective route offers an attractive starting point for mildly hypotensive alimentary or even veterinary products. Because the present route also includes a surprisingly simple purification step, the possibilities for blood pressure lowering concentrated dietary supplements are also enlarged.

By the proline specific endo protease used according to the invention is meant the polypeptide as mentioned in claims 1-5, 11 and 13 of WO 02/45524. Therefore this proline specific endo protease is a polypeptide which has proline specific endoproteolytic activity, selected from the group consisting of:

(a) a polypeptide which has an amino acid sequence which has at least 40% amino acid sequence identity with amino acids 1 to 526 of SEQ ID NO:2 or a fragment thereof;
(b) a polypeptide which is encoded by a polynucleotide which hybridizes under low stringency conditions with (i) the nucleic acid sequence of SEQ ID NO:1 or a fragment thereof which is at least 80% or 90% identical over 60, preferably over 100 nucleotides, more preferably at least 90% identical over 200 nucleotides, or (ii) a nucleic acid sequence complementary to the nucleic acid sequence of SEQ ID NO:1. The SEQ ID NO:1 and SEQ ID NO:2 as shown in WO 02/45524. Preferably the polypeptide is in isolated form.

The preferred polypeptide used according to the present invention has an amino acid sequence which has at least 50%, preferably at least 60%, preferably at least 65%, preferably at least 70%, more preferably at least 80%, even more preferably at least 90%, most preferably at least 95%, and even most preferably at least about 97% identity with amino acids 1 to 526 of SEQ ID NO: 2 or comprising the amino acid sequence of SEQ ID NO:2.

Preferably the polypeptide is encoded by a polynucleotide that hybridizes under low stringency conditions, more preferably medium stringency conditions, and most preferably high stringency conditions, with (i) the nucleic acid sequence of SEQ ID NO:1 or a fragment thereof, or (ii) a nucleic acid sequence complementary to the nucleic acid sequence of SEQ ID NO: 1.

The term "capable of hybridizing" means that the target polynucleotide of the invention can hybridize to the nucleic acid used as a probe (for example, the nucleotide sequence set forth in SEQ. ID NO: 1, or a fragment thereof, or the complement of SEQ ID NO: 1) at a level significantly above background. The invention also includes the use according to the claims of polynucleotides that encode the proline specific endoprotease of the invention, as well as nucleotide sequences which are complementary thereto. The nucleotide sequence may be RNA or DNA, including genomic DNA, synthetic DNA or cDNA. Preferably, the nucleotide sequence is DNA and most preferably, a genomic DNA sequence. Typically, a polynucleotide of the invention comprises a contiguous sequence of nucleotides which is capable of hybridizing under selective conditions to the coding sequence or the complement of the coding sequence of SEQ ID NO: 1. Such nucleotides can be synthesized according to methods well known in the art.

A polynucleotide of the invention can hybridize to the coding sequence or the complement of the coding sequence of SEQ ID NO:1 at a level significantly above background. Background hybridization may occur, for example, because of other cDNAs present in a cDNA library. The signal level generated by the interaction between a polynucleotide of the invention and the coding sequence or complement of the coding sequence of SEQ ID NO: 1 is typically at least 10 fold, preferably at least 20 fold, more preferably at least 50 fold, and even more preferably at least 100 fold, as intense as interactions between other polynucleotides and the coding sequence of SEQ ID NO: 1. The intensity of interaction may be measured, for example, by radiolabelling the probe, for example with 32P. Selective hybridization may typically be achieved using conditions of low stringency (0.3M sodium chloride and 0.03M sodium citrate at about 40°C), medium stringency (for example, 0.3M sodium chloride and 0.03M sodium citrate at about 50°C) or high stringency (for example, 0.3M sodium chloride and 0.03M sodium citrate at about 60°C).

The UWGCG Package provides the BESTFIT program which may be used to calculate identity (for example used on its default settings).

The PILEUP and BLAST N algorithms can also be used to calculate sequence identity or to line up sequences (such as identifying equivalent or corresponding sequences, for example on their default settings).

Software for performing BLAST analyses is publicly available through the National Center for Biotechnology Information (http://www.ncbi.nlm.nih.gov/). This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold. These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix alignments (B) of 50, expectation (E) of 10, M=5, N=4, and a comparison of both strands.

The BLAST algorithm performs a statistical analysis of the similarity between two sequences. One measure of similarity provided by the BLAST algorithm is the smallest sum probability (P(N)), which provides an indication of the probability by which a match between two nucleotide or amino acid sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001.

The strains of the genus Aspergillus have a food grade status and enzymes derived from these micro-organisms are known to be from an unsuspect food grade source. According to another preferred embodiment, the enzyme is secreted by its producing cell rather than a non-secreted, socalled cytosolic enzyme. In this way enzymes can be recovered from the cell broth in an essentially pure state without expensive purification steps. Preferably the enzyme has a high affinity towards its substrate under the prevailing pH and temperature conditions.

Description of the Figures

**[0053]**

　　　　Figure 1: A graphic representation of the pH optimum of the A. niger derived prolyl endoprotease
　　　　Figure 2: Specificity profile of the A. niger derived prolyl endoprotease
　　　　Figure 3: SDS-PAGE of intact ovalbumine and a synthetic 27-mer peptide after incubation with chromatographically purified *A. niger* derived proline specific endoprotease.

Materials and Methods

Materials.

Edible potassium caseinate spray (88%) was obtained from DMV International, The Netherlands. Synthetic chromogenic peptides were obtained from either Pepscan Systems B.V. The Netherlands; or from Bachem, Switzerland.

Proline- specific endoprotease from *A. niger.*

Overproduction of the proline specific endoprotease from Aspergillus niger was accomplished as described in WO 02/45524. The activity of the enzyme was tested on the synthetic peptide Z-Gly-Pro-pNA at 37 degrees C in a citrate/disodium phosphate buffer pH 4.6. The reaction product was monitored spectrophotometrically at 405 nM. A unit is defined as the quantity of enzyme that liberates 1 $\mu$mol of p-nitroanilide per minute under these test conditions.

Chromatographic purification of the A. niger derived endoprotease

The culture broth obtained from an overproducting A. niger strain was used for chromotograhpic purification of the protease to remove any contaminating endo- and exoproteolytic activities. To that end the fermentation broth was first centrifuged to remove the bulk of the fungal mass and the supernatant was then passed through a number of filters with decreasing pore sizes to remove all cell fragments. Finally, the ultrafiltrate obtained was diluted ten times in 20 millimol/liter sodium acetate pH 5.1 and applied on a Q-Sepharose FF column. Proteins were eluted in a gradient from 0 to 0.4 moles/liter NaCl in 20 millimol/liter sodium acetate pH 5.1. Peak fractions displaying activity towards the cleavage of Z-Gly-Pro-pNA were collected and pooled, according to the protocol described in World Journal of Microbiology & Biotechnology 11, 209 - 212 (1995), but under slightly modified assay conditions. Taking the acid pH optimum of the A. niger derived proline-specific endoprotease into account, the enzyme assay was carried out at pH 4.6 in a citrate/diphosphate buffer at 37°C. Pooling of the active fractions followed by concentration finally yielded a preparation which showed only a single band on SDS-PAGE and one peak on HP-SEC. Further analysis by hydrophobic interaction chromatography confirmed the purity of the enzyme preparation obtained.

LC/MS/MS analysis used in the detection of IPP, LPP and VPP.

HPLC using an ion trap mass spectrometer (Thermoquest®, Breda, the Netherlands) coupled to a P4000 pump (Thermoquest®, Breda, the Netherlands) was used in quantification of the peptides of interest, among these the tripeptides IPP, LPP and VPP, in the enzymatic protein hydrolysates produced by the inventive enzyme mixture. The peptides formed were separated using a Inertsil 3 ODS 3, 3 $\mu$m, 150*2.1 mm (Varian Belgium, Belgium) column in combination with a gradient of 0.1 % formic acid in Milli Q water (Millipore, Bedford, MA, USA; Solution A) and 0.1% formic acid in acetonitrile (Solution B) for elution. The gradient started at 100% of Solution A, kept here for 5 minutes, increasing linear to 5 % B in 10 minutes, followed by linear increasing to 45% of solution B in 30 minutes and immediately going to the beginning conditions, and kept here 15 minutes for stabilization. The injection volume used was 50 microliters, the flow rate was 200 microliter per minute and the column temperature was maintained at 55°C. The protein concentration of the injected sample was approx. 50 micrograms/milliliter.

Detailed information on the individual peptides was obtained by using dedicated MS/MS for the peptides of interest, using optimal collision energy of about 30 %. Quantification of the individual peptides was performed using external calibration, by using the most abundant fragment ions observed in MS/MS mode.

The tripeptide LPP (M=325.2) was used to tune for optimal sensitivity in MS mode and for optimal fragmentation in MS/MS mode, performing constant infusion of 5 $\mu$g/ml, resulting in a protonated molecule in MS mode, and an optimal collision energy of about 30 % in MS/MS mode, generating a B- and Y-ion series.

Prior to LC/MS/MS the enzymatic protein hydrolysates were centrifuged at ambient temperature and 13000 rpm for 10 minutes, filtered through a 0.22 $\mu$m filter and the supernatant was diluted 1:100 with MilliQ water.

Kjeldahl Nitrogen

Total Kjeldahl Nitrogen was measured by Flow Injection Analysis. Using a Tecator FIASTAR 5000 Flow Injection System equipped with a TKN Method Cassette 5000-040, a Pentium 4 computer with SOFIA software and a Tecator 5027 Autosampler the ammonia released from protein containing solutions was quantitated at 590 nm. A sample amount corresponding with the dynamic range of the method (0.5-20 mg N/l) is placed in the digestion tube together with 95-97% sulphuric acid and a Kjeltab subjected to a digestion program of 30 minutes at 200 degrees C followed by 90 minutes at 360 degrees C. After injection in the FIASTAR 5000 system the nitrogen peak is measured from which the amount of protein measured can be inferred.

Amino acid analysis

A precisely weighed sample of the proteinaceous material was dissolved in dilute acid and precipitates were removed by centrifugation in an Eppendorf centrifuge. Amino acid analysis was carried out on the clear supernatant according to the PicoTag method as specified in the operators manual of the Amino Acid Analysis System of Waters (Milford MA, USA). To that end a suitable sample was obtained from the liquid, then dried and subjected to vapour phase acid hydrolysis and derivatised using phenylisothiocyanate. The various derivatised amino acids present were quantitated using HPLC methods and added up to calculate the total level of free amino acids in the weighed sample. The amino acids Cys and Trp are not included in the data obtained in this analysis.

The Degree of Hydrolysis (DH) of the protein hydrolysate was measured using a rapid OPA test and calculated as described (Nielsen et al, JFS, Vol 66, NO 5, 642-646, 2001).

Molecular weight distribution of peptides and proteins present in hydrolysates.

Analysis of the peptide size distribution of protease treated protein samples was done on an automated HPLC system equiped with a high pressure pump, injection device able to inject 10-100 $\mu$l sample and a UV detector able to monitor the column effluent at 214 nm.

The column used for this analysis was a Superdex Peptide HR 10/300 GL (Amersham) equilibrated with 20 mM Sodium Phosphate / 250 mM Sodium Chloride pH 7.0 buffer. After injecting a sample (typically 50 $\mu$l) the various components were eluted from the column with buffer in 90 min at a flow rate of 0.5 ml/min. The system was calibrated using a mixture of cytochrome C (Mw 13 500 Da), aprotinin (Mw 6510 Da) and tetraglycine (Mw 246 Da) as molecular weight markers.

Example 1

The enzyme as obtained from *A. niger* represents a new class of proline specific enzymes.

From the entire coding sequence of the *A. niger* derived proline specific endoprotease as provided in WO 02/45524 a protein sequence of 526 amino acids can be determined. The novelty of the enzyme was confirmed by BLAST searches of databases such as SwissProt, PIR and trEMBL. To our surprise, no clear homology could be detected between the *A. niger* enzyme and the known prolyl oligopeptidases. Closer inspection of the amino acid sequence, however, revealed low but significant homology to Pro-X carboxypeptidases (EC3.4.16.2), dipeptidyl aminopeptidases I (EC3.4.14.2), and thymus specific serine protease. All of these enzymes have been assigned to family S28 of serine peptidases. Also the GxSYxG configuration around the active site serine is conserved between these enzymes and the *A. niger* derived endoprotease. Additionally, members of family S28 have an acidic pH optimum, have specificity for cleaving at the carboxy-terminal side of proline residues and are synthesized with a signal sequence and propeptide just like the *A. niger* derived proline specific endoprotease. Also the size of the *A. niger* enzyme is similar to those the members of family S28. Therefore, the *A. niger* proline specific endoprotease appears to be a member of family S28 of serine proteases rather than the S9 family into which most cytosolic prolyl oligopeptidases including the enzyme obtained from *Flavobacterium meningosepticum* have been grouped. On the basis of these structural and physiological features we have concluded that the *A. niger* enzyme belongs to the S28 rather than the S9 family of serine proteases. An additional feature that discriminates the *A. niger* derived enzyme from the prolyl oligopeptidases belonging to the S9 family is the fact that, unlike the cytosolic prolyl endoproteases belonging to the latter family, the newly identified *A. niger* enzyme is secreted into the growth medium. This is the first report on the isolation and characterization of a member of family S28 from a lower eukaryote.

### Example 2

The pH and temperature optima of the proline specific endoprotease as obtained from *A. niger* .

To establish the pH optimum of the *A. niger* derived proline specific endoprotease, buffers with different pH values were prepared. Buffers of pH 4.0 - 4.5 - 4.8 - 5.0 - 5.5 and 6.0 were made using 0.05 mol/l Na-acetate and 0.02 M CaCl2; buffers of pH 7.0 and 8.0 were made using 0.05 M Tris/HCl buffers containing 0.02 M CaCl2. The pH values were adjusted using acetic acid and HCl respectively. The chromogenic synthetic peptide Z-Gly-Pro-pNA was used as the substrate. The buffer solution, the substrate solution and the prolyl endoprotease pre-dilution (in an activity of 0.1 U/mL), were heated to exactly 37.0°C in a waterbath. After mixing the reaction was followed spectrophotometrically at 405 nm at 37.0°C for 3.5 min, measuring every 0.5 min. From the results shown in Figure 1 it is clear that the *A. niger* derived proline specific endoprotease has a pH optimum around 4.

Also the temperature optimum of the prolyl endoprotease was established. To that end the purified enzyme preparation was incubated in 0.1 mol/l Na-acetate containing 0.02 mol/l CaCl2 at pH 5.0 for 2 hours at different temperatures using Caseine Resorufine (Roche version 3) as the substrate and enzyme activity was quantified by measuring at 574 nm. According to the results obtained the proline specific endoprotease from *A. niger* has a temperature optimum around 50 degrees C.

### Example 3

The specificity and purity of the *A. niger* derived proline specific endoprotease.

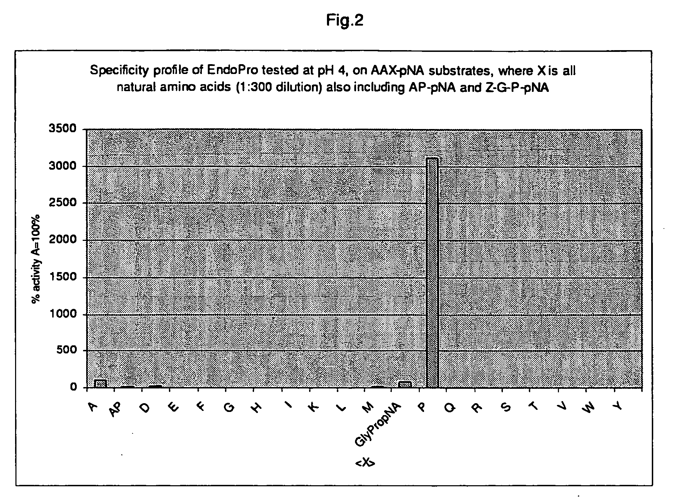

Crude as well as chromatographically purified enzyme samples as obtained from an A. niger strain containing multiple copies of the expression cassette (cf WO 02/45524) were tested against a set of chromogenic peptide substrates to establish the specificity of the encoded endoprotease. Using different sets of chromogenic peptides the presence of possible contaminating enzyme activities was established. The endoproteolytic specificity of the encoded endoprotease was tested on different Ala-Ala-XpNA (AAXpNA) substrates. In this context "X" refers to the different natural amino acid residues and "pNA" to p-Nitroanilide. Cleavage of the peptide bond between the "X" residue and the pNA moiety of the molecule, causes a color change that can be monitored by an increase in the light absorbance at lambda= 405 or 410 nm.The AAXpNA substrate. To that end stock solutions of AAX-pNA substrates (150 mmol/l) were diluted 100X in 0.1 M acetate buffer pH 4.0 containing 20 CaCl2. The 10 minutes kinetic measurements in a TECAN Genios MTP Reader (Salzburg, Vienna) at 405nm recorded the increases in optical density that via data processing in Excel yielded the picture shown in Figure 2. From the result it is clear that the A. niger derived endoprotease is highly specific for prolyl peptide bonds with a side activity towards alanyl bonds. Crude and chromatographically purified preparations showed similar activity profiles.

A possible contamination of the A. niger derived endoprotease with foreign enzymatic activities leads a number of undesirable side reactions. For example, the presence of exoproteases such as carboxypeptidases or aminopeptidases, results in peptide preparations having increased levels of free amino acids. These extra free amino acids dilute the relative concentrations of the bio-active peptides present and, moreover, impart brothy off tastes as the result of increased Maillard reactions. Should serious levels of contaminating endoproteases be present in the preparation of the overexpressed proline specific endoprotease, cleavage sites at other positions than carboxyterminal of proline or alanine residues are introduced. Such extra cleavage sites generate extra peptides hereby also diluting the concentration of bio-active peptides having carboxyterminal proline residues. To minimise all these undesirable side reactions, the use of an essentially pure proline specific protease is preferred. Essentially pure meaning that the activity of contaminating endoproteases as well as contaminating exopeptidases under the incubation conditions used are minimal or preferably absent. The following testing procedure was devised to quantitate such contaminating endo- and exopeptidases activities.

The basis for the testing procedure is formed by a collection of various selective chromogenic peptides. Because only proline specific oligo- and endoproteases can release pNA from peptide Z-AAAP-pNA, this particular peptide was used to quantitate the desired proline specific endoproteolytic activity. Because many endoproteases can release pNA from peptides Z-AAAF-pNA and Z-AAAR-pNA, these two peptides were used to quantitate contaminating, non-proline specific endoproteolytic activity.

Because many aminopeptidases can efficiently release Phe and Gln from peptide substrates, the chromogenic peptides Q-pNA and V-pNA were used to quantitate contaminating aminopeptidase activities.

Because many carboxypeptidases can release Phe and Arg residues from peptide substrates, peptides containing these residues were selected to quantitate contaminating carboxypeptidase activities. However, no suitable chromogenic groups are available for measuring carboxypeptidase activities so that an alternative method using the synthetic peptides Z-AF and Z-AR had to be developed. This alternative method is provided underneath. In all the synthetic peptides used "Z" represents benzyloxycarbonyl and "pNA" the chromophore para-nitroanilide. All chromogenic peptides were obtained from Pepscan (Lelystad, The Netherlands). Peptides Z-AF and Z-AR were purchased from Bachem (Switserland). All incubations were carried out at 40°C. Diluted enzyme preparations were recalculated to the concentration of the commercial product.

To illustrate the levels of the various enzyme activities that are regularly present in industrially available enzyme preparations, in this Example we describe the testing of three commercial enzyme preparations for their various proteolytic activities i.e.: Flavourzyme 1000L Batch HPN00218 (Novozymes), Sumizyme FP (Shin Nihon, Japan) and Corolase LAP Ch.: 4123 (AB Enzymes, UK). Both Flavourzyme and Sumizyme FP are known to be complex enzyme preparations that contain several aminopeptidolytic enzyme activities besides non-specified endoproteolytic and carboxypeptidolytic activities. Corolase LAP represents a relatively pure, cloned and overexpressed leucine aminopeptidase activity from Aspergillus.

Measuring AminoPeptidase activities

Stock solutions of 150mmol/l of F-pNA and Q- pNA in 100% DMSO were diluted 80 times in 0.1 M BisTris buffer pH 6 to make a 3.75 mmol/l F-pNA+ Q-pNA -substrate solution containing F-pNA and Q-pNA in a 1:1 ratio. A 200 $\mu$l aliquot of this aminopeptidase substrate solution was pipetted into separate wells of a microtiterplate (MTP) The MTP is pre-incubated at 40°C in a Tecan Genios MTP (Salzburg, Vienna) running under Magellan4 software. The reaction was started by adding 50 $\mu$l of the appropriate enzyme solution so that the incubations took place at a substrate concentration of 3mM. Typically a 1:50 dilution of the liquid enzyme samples Flavourzyme, Corolase LAP and proline-specific endo-protease was used. Of the dry Sumizyme FP product a 1 % solution was used.
The yellow color as measured at 405nm by the Tecan Genios MTP developing as the result of cleavage of the amino acid-pNA bond was followed for at least 20 kinetic cycles (about 10 minutes). The software generated the data obtained as $OD_{405}$/min.

Measuring Proline Specific Endoprotease activity.

This measurement was carried out essentially the same as the aminopeptidase assay but in this case Z-AAAP-pNA was used as the only substrate in a final concentration of 3mmol/l. This substrate was solubilized by heating a suspension in pH 6 buffer to 50-55° C resulting in a clear solution at room temperature. Measurements were carried out at 40°C.
Typically a 1:50 dilution of the liquid enzyme samples Flavourzyme and Corolase LAP were used. Sumizyme FP was used in a 1% solution. The proline specific endo-protease was typically used in a 1:5000 dilution.
The software generated the data as $OD_{405}$/min.

Measuring Contaminating Non-Proline Specific Endoprotease activities.

Also this measurement was carried out essentially the same as described for the aminopeptidase assay but in this test Z-AAAF-pNA and Z-AAAR-pNA in a 1:1 ratio and in a final concentration of 3mmol/l were used as the substrate. The substrate Z-AAAF-pNA turned out to be poorly soluble under the pH 6.0 test conditions used but a test incubation with subtilisin resulted in a rapid solubilisation of the substrate concomitantly with the pNA release. Measurements were carried out at 40°C. However, to compensate for this poor solubility the MTP reader was programmed to shake in between the kinetic cycles.
Again the software generated the data as $OD_{405}$/min.

Measuring Contaminating Carboxypeptidase activities

Because no sensitive chromogenic peptides are available to measure carboxypeptidase activities, a method was used based on a Boehringer protocol for quantitating Carboxypeptidase C. Two 150mmol/l stock solutions in ethanol of Z-A-F and Z-A-R were diluted 80 times in 0.1 mol/l BisTris buffer pH 6 to make a 3.75 mmol/l Z-A-F + Z-A-R substrate solution containing Z-A-F and Z-A-R in a 1:1 ratio. Then 200 μl of the substrate solution was pipetted into an eppendorf vial and pre-incubated at 40° C. The reaction was started by adding 50 μl of an appropriate enzyme dilution. Typically a 1:50 dilution is used of Flavourzyme and Corolase LAP and the proline specific endoprotease. A 1 % solution was used for Sumizym FP. After 5 minutes the reaction was stopped by adding 250 μl of ninhydrine reagent. Ninhydrine reagent was made of 400mg ninhydrine (Merck) and 60 mg hydrindantin dissolved 15 ml DMSO, to which 5 ml of 4.0 mol/l lithium acetate buffer pH 5.2 was added. The 4.0 mol/l lithium acetate buffer was made by dissolving LiOH (Sigma) after which the pH of the solution was adjusted to pH 5.2 using glacial acetic acid (Merck).
After stopping the reaction, each sample was heated for 15 minutes at 95°C to facilitate the color formation and subsequently diluted 10 times with pure ethanol. The color formed was measured at 578nm in an Uvikon spectrophotometer. Blanks were made in the same manner as the activity samples, but ninhydrin reagent and enzyme addition were reversed. To quantitate the amount of free amino acids generated by the carboxypeptidase activity, the amino acid L-phenylalanine was used to create a calibration curve. Solutions in buffer pH 6 containing 0.1875, 0.375, 0.75, 1.5 and 3.0 mmol/l of L-phenylalanine (Sigma) were treated in the same manner as the samples, i.e. 250μl in a vial. From the OD578 values obtained, a curve was constructed in Excel. The concentrations of the free amino acids present in the samples containing the Z-A-F and Z-A-R substrates were calculated using this curve. From the values obtained the carboxy-peptidase activity was calculated in micromoles per minute per the amount of enzyme tested.

Calculation of activity ratios

To establish the suitability of various enzyme preparations for the process according to the invention, quotients of the relevant enzyme activities were calculated. In the MTP reader based assays, enzyme activities are characterised by pNA release over time i.e. as $\Delta OD_{405}$/min. Quotients of enzyme activities obtained by the MTP reader were calculated by simply dividing the $\Delta OD$/min values obtained for identical quantities of enzyme.

However in case of the carboxy-peptidase assay, an OD is generated that cannot be compared directly to the $\Delta OD$/min generated by the MTP-pNA based assays. Here the OD measured was first converted to μmol amino acid released per min (μmol/min). Then the $\Delta OD$/min of pNA released was converted into μmol/min. To that end a calibration curve was generated in the MTP reader in which dilutions of pure pNA (Sigma) 0.25, 0.125, 0.0625, 0.0312 and 0.015 mmol/l and 250μl per well were measured. From the data obtained a calibration curve was constructed in Excel. From this calibration curve the $\Delta OD$/min was converted into μmol/min so that the pNA based measurements could be compared with the ninhydrin based measurements.

On the basis of the data generated in the above-mentioned tests, the various enzyme preparations used were characterised in terms of desirable proline (and alanine) specific activities and contaminating endoprotease, aminopeptidase and carboxypeptidase activities. The data on the proline specific oligo- or endoproteolytic activities present in each enzyme preparation are shown in Table 1 in the column "Prol Spec Activy". The data on the contaminating aminopeptidase activities (AP/Prol Spec Act), the contaminating carboxypeptidase (CPD/Prol Spec Act) and the contaminating endoproteolytic activities (Endo/Prol Spec Act) are shown relative to the proline specific activities present. The level of the contaminating aminopeptidase activity relative to the contaminating carboxypeptidase activity present in each preparation is shown as (AP/CPD).
Evident is that none of the commercial enzyme preparations tested contains any significant proline specific oligo- or endoproteolytic activity. Furthermore all commercial enzyme preparations tested contain significant contaminating endo- or exoproteolytic activities.

Table 1: Levels of desired (proline and alanine specific) endoprotease activities relative to the levels of contaminating endo- and exo proteolytic activities.

| | Prol Spec Activity* | CPD/Prol spec act | AP/Prol spec act | Endo/Prol spec act | AP/ CPD |
|---|---|---|---|---|---|
| | | | | | |
| Sumizyme | 0.004 | 21.7 | 1.2 | 1.7 | 0.06 |
| Flavourzyme | 0.0007 | 253.5 | 25.6 | 35.5 | 0.10 |

(continued)

| | Prol Spec Activity* | CPD/Prol spec act | AP/Prol spec act | Endo/Prol spec act | AP/ CPD |
|---|---|---|---|---|---|
| Corolase LAP | 0.0 | | | | 0.74 |
| Prol spec A.niger | 100 | 0.005 | 0.00001 | 0.000004 | 0.00 |
| * Sumizyme was measured in a 1% solution, Flavourzyme and Corolase as a 1:50 dilution. Prol specific activity as obtained from *A. niger* was measured as 1:5000 dilution. Data were then calculated to the activity present in the product as provided. | | | | | |

Example 4

The *A. niger* derived proline specific endoprotease can hydrolyse large proteins as well as small peptides and is thus a true endoprotease.

Owing to a specific structural feature, prolyl oligopeptidases belonging to the S9 family cannot digest peptides larger than 30 amino acids. This limitation is an obvious disadvantage for an enzyme, which is meant to hydrolyse as quickly and as efficiently as possible different proteins. To see if the *A. niger* derived proline specific endoprotease exhibits the same limitations with respect to the size of the substrate molecule, we have incubated the chromatographically purified prolyl endopeptidase from A. *niger* with a small synthetic peptide and with the large ovalbumine molecule and have analysed the hydrolysis products formed by SDS-PAGE.

The synthetic peptide used was a 27-mer of the sequence NH2-FRASDNDRVIDPGKVETLTIRRLHIPR-COOH and was a gift of the Pepscan company (Lelystad,The Netherlands). As shown by its amino acid sequence, this peptide contains 2 proline residues, one in the middle and one at the very end of the peptide.

The intact ovalbumine molecule (Pierce Imject, vials containing 20mg freeze dried material) consists of 385 amino acids with a molecular weight of 42 750 Da. This molecule contains 14 proline residues, one of which is located at the ultimate C-terminal end of the molecule and cannot be cleaved by a proline specific endoprotease.

Ovalbumin and the oligopeptide were separately incubated at 50°C with the purified *A. niger* derived proline specific endoprotease. At several time intervals samples were taken which were the analysed using SDS-PAGE.

A chromatographically purified A. niger derived proline specific endoprotease with an activity of 4.5 units/ml was diluted 100-fold with 0.1 M acetate buffer pH 4 containing 20mM CaCl2. The ovalbumine was dissolved in acetate buffer pH 4 to a concentration of 1 mg/ml (22$\mu$M). The 27-mer was dissolved in the same buffer to reach a concentration of 0.48 mg/ml (152$\mu$M). The molarity of the ovalbumine and the 27-mer solution was chosen in such a way that both solutions contained the same molarity in cleavable proline residues. Ovalbumine contains 13 potential proline cleavage sites, whereas the 27-mer peptide has only two. Of both substrate solutions 0.5 ml was incubated with 10$\mu$l (0.45milliU) of the enzyme solution in an Eppendorf thermomixer at 50°C. At several time intervals 10$\mu$l samples were withdrawn from the incubation mixture and kept at 20°C until SDS-PAGE. All materials used for SDS-PAGE and staining were purchased from Invitrogen. Samples were prepared using LDS buffer according to manufacturers instructions and separated on 12% Bis-Tris gels using MES-SDS buffer system according to manufacturers instructions. Staining was performed using Simply Blue Safe Stain (Collodial Coomassie G250).

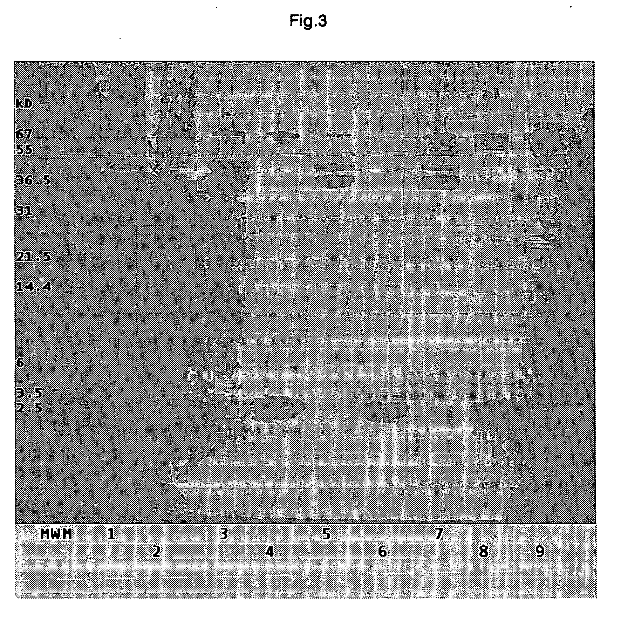

As can be seen in Figure 3 ovalbumine is cleaved by the *Aspergillus* derived enzyme into a discrete band of about 35 to 36kD in the first 4.75 hours of incubation (lane 3). Prolonged incubation periods result in further breakdown to smaller products of various molecular weights (lane 7).

The 27-mer peptide is also broken down, as judged by the more faint bands in lanes 4, 6 and 8 as compared to lane 2. The very small molecular weight shift of the product (compare lanes 9 and 8) is most likely due to cleaving of the arginine residue at the carboxylic end of the peptide. The difference is about 200D (measured using Alphalmager 3.3d software on an Alphalmager 2000 system) and arginine has a MW of 174. This small molecular weight shift is probably the first step in the breakdown of the peptide.

The further decay of the product can only be seen by the decrease in intensity of the band on the SDS gel. The products of further decay are not visible, as in gel staining of components with a MW of about 1000 is not possible with Coomassie Brilliant Blue.

From this experiment it can be concluded that, unlike the known prolyl oligopeptidases belonging to the S9 family, the *A. niger* derived proline specific endoprotease has no specific preference for cleaving small sized peptides over much larger proteins. As such the *A. niger* derived enzyme represents a true endoprotease and a preferred enzyme to hydrolyse different types of proteins. This finding led to the surprising use of the enzyme as illustrated in the following Example.

Example 5

Incubating potassium caseinate with the proline specific endoprotease from A. niger quickly yields IPP and LPP but no VPP.

In this experiment the overproduced and essentially pure proline specific endoprotease from A. niger was incubated with potassium caseinate to test the liberation of the ACE inhibiting peptides IPP, VPP as well as LPP. The endoprotease used was essentially pure meaning that no significant endoproteolytic activity other than the endoproteolytic activity inherent to the pure proline specific endoprotease (i.e. carboxyterminal cleavage of proline and alanine residues) is present. Furthermore the enzyme preparation is devoid of significant exoproteolytic activities as described in Example 3.

To limit sodium intake as the result of the ingestion of ACE inhibiting peptides as much as possible, potassium caseinate was used as the substrate in this incubation.

The caseinate was suspended in water of 65 degrees C in a concentration of 10% (w/w) protein after which the pH was adjusted to 6.0 using phosphoric acid. Then the suspension was cooled to 55 degrees C and the A. niger derived proline specific endoprotease was added in a concentration of 4 units/gram of protein (see Materials & Methods section for unit definition). Under continuous stirring this mixture was incubated for 24 hours. No further pH adjustments were carried out during this period. Samples were taken after 1, 2, 3, 4, 8 and 24 hours of incubation. Of each sample enzyme activity was terminated by immediate heating of the sample to 90 degrees C for 5 minutes. After cooling down the pH of each sample was quickly lowered to 4.5 using phosphoric acid after which the suspension was centrifuged for 5 minutes at 3000 rpm in a Hereaus table top centrifuge. The completely clear supernatant was used for LC/MS/MS analysis to quantify the peptides VPP, IPP, LPP, VVVPP and VVVPPF in the supernatant (see Materials & Methods section).

Bovine milk casein incorporates a number of different proteins including beta-casein and kappa-casein. According to the known amino sequences beta-casein encompasses the ACE inhibitory tripeptides IPP, VPP and LPP. In beta-casein IPP is contained in the sequence $-P_{71}-Q_{72}-N_{73}-I_{74}-P_{75}-P_{76}-$, VPP is contained in the sequence $-P_{81}-V_{82}-V_{83}-V_{84}-P_{85}-P_{86}-$ and LPP is contained in the sequence $-P_{150}-L_{151}-P_{152}-P_{153}-$. Kappa-casein, which is present in acid precipitated caseinate preparations in a molar concentration of almost 50% of the beta-casein concentration, encompasses IPP only. In kappa-casein IPP is contained in the sequence $-A_{107}-I_{108}-P_{109}-P_{111}-$. The other protein constituents of casein do not contain either IPP, VPP or LPP.

Tables 2 and 3 show the concentrations of the peptides present in the acidified and centrifuged supernatants as calculated per gram of potassium caseinate added to the incubation mixture. As shown in Table 2, IPP reaches its maximal concentration after 1 hour of incubation. Beyond that the IPP concentration does not increase any further. The formation of the pentapeptide VWPP shows the same kinetics as the generation of IPP. As theoretically expected, the molar yield of VWPP is similar to the molar yield of the LPP peptide. The yield of both LPP and VVVPP reach almost 60% of what would be theoretically feasible. The fact that the maximum concentration of LPP is reached only after 3 hours of incubation suggests that cleavage of that particular part of the beta-caseine molecule is perhaps somewhat more difficult. In contrast with VVVPP, the hexapeptide VVVPPF is not formed at all. This observation suggests that the proline specific endoprotease efficiently cleaves the -P-F- bond hereby generating VVVPP. The tripeptide IPP is formed immediately but its molar yield is not more than about a third of the maximal molar yield of either VWPP or LPP. As the IPP tripeptide is contained in both beta-caseine as in kappa-caseine, this outcome is unexpected. A likely explanation for this observation is that the proline specific protease can generate IPP but from the kappa-caseine moiety of the caseinates only. In view of the relevant amino acid sequence of kappa-caseine this suggests that the $-A_{107}-I_{108}-$ peptide bond is cleaved by the alanine-specific activity of the enzyme. If true, the amount of IPP liberated reaches approximately 40 % of the quantity that is present in kappa-casein, but not more than about 10% of the IPP that is theoretically present in beta plus kappa casein. This cleavage mechanism for the release of IPP also explains why VPP cannot be formed from its precursor molecule VWPP: the required endoproteolytic activity is simply not present within the A. niger derived enzyme preparation used.

Table 2 Molar peptide contents of acidified supernatants calculated per gram of protein added

| micromole/gram protein | IPP | LPP | VPP | VVVPP | VVVPPF |
|---|---|---|---|---|---|
| K-cas 1 hr | 2.8 | 4.2 | < 0.2 | 8.4 | < 0.2 |
| K-cas 2 hrs | 2.6 | 6.1 | < 0.2 | 9.1 | < 0.2 |
| K-cas 3 hrs | 2.6 | 8.4 | < 0.2 | 9.1 | < 0.2 |
| K-cas 4 hrs | 2.3 | 8.0 | < 0.2 | 8.3 | < 0.2 |
| K-cas 8 hrs | 2.1 | 9.4 | < 0.2 | 7.2 | < 0.2 |
| K-cas 24 hrs | 2.0 | 9.5 | 0.4 | 5.5 | < 0.2 |

Table 3 Peptide concentrations in acidified supernatants calculated in mg/g protein added

| milligram/gram protein | IPP | LPP | VPP | VVVPP | VWPPF |
|---|---|---|---|---|---|
| K-cas 1 hr | 0.9 | 1.4 | < 0.05 | 4.3 | < 0.05 |
| K-cas 2 hrs | 0.8 | 2.0 | < 0.05 | 4.6 | < 0.05 |
| K-cas 3 hrs | 0.8 | 2.7 | < 0.05 | 4.6 | < 0.05 |
| K-cas 4 hrs | 0.8 | 2.6 | < 0.05 | 4.2 | < 0.05 |
| K-cas 8 hrs | 0.7 | 3.0 | < 0.05 | 3.6 | < 0.05 |
| K-cas 24 hrs | 0.7 | 3.1 | 0.1 | 2.8 | < 0.05 |

Example 6

Incorporation of an acid casein precipitation step results in a 5-fold concentration of ACE inhibiting peptides

As described in Example 5, potassium caseinate in a concentration of 10% (w/w) protein was subjected to an incubation with the A. niger derived proline specific endoprotease at pH 6.0. After various incubation periods samples were heated to stop further enzyme activity after which the pH was lowered to 4.5 to minimise casein solubility. Non soluble casein molecules were removed by a low speed centrifugation. In Tables 2 and 3 we have provided concentrations of ACE inhibiting peptides calculated on the basis of the starting concentration of 10% protein. However, as the result of the acidification and the subsequent centrifugation step, a large proportion of the protein added has been removed. To take these reduced protein contents of the acidified supernatants into account, nitrogen (Kjeldahl) analyses were carried out. According to the latter data the various supernatants were found to contain the protein levels shown in Table 4.

Table 4 Protein contents of acidified supernatants

| Sample | Protein content (grams/liter) |
|---|---|
| K-cas 1 hr | 21 |
| K-cas 2 hrs | 27 |
| K-cas 3 hrs | 30 |
| K-cas 4 hrs | 34 |
| K-cas 8 hrs | 40 |
| K-cas 24 hrs | 48 |

Taking these data into account, we have recalculated the concentration of the ACE inhibiting peptides present in each supernatant but this time using their actual protein contents. These recalculated data are shown in Table 5.

Table 5 Peptide concentrations in acidified supernatants calculated per gram of protein present.

| milligram/gram protein | VPP | IPP | LPP | VVVPP | VWPPF |
|---|---|---|---|---|---|
| K-cas 1 hr | 0.1 | 4.8 | 7.1 | 22.5 | < 0.05 |
| K-cas 2 hr | 0.1 | 3.4 | 8.0 | 18.9 | < 0.05 |
| K-cas 3 hr | 0.1 | 3.1 | 10.0 | 17.0 | < 0.05 |
| K-cas 4 hr | 0.1 | 2.4 | 8.5 | 13.7 | < 0.05 |
| K-cas 8 hr | 0.1 | 1.9 | 8.4 | 10.0 | < 0.05 |
| K-cas 24 hr | 0.3 | 1.5 | 7.1 | 6.4 | < 0.05 |

Comparison of the data presented in Tables 3 and 5 clearly shows that the simple acidification step followed by an industrially feasible decantation, filtration or low speed centrifugation step results in a 5-fold increase in the concentration of the specific ACE inhibiting peptides.

Example 7

Identification of the novel and potent ACE inhibiting tripeptides MAP and ITP in concentrated casein hydrolysates

To facilitate a more thorough analysis of bio-active peptides present, the casein hydrolysate obtained by the digestion with pure A. niger derived proline specific endoprotease and purified by acid precipitation was prepared on a preparative scale. To that end 3000 grams of potassium caseinate was suspended in 25 liters of water of 75 degrees C. After a thorough homogenisation the pH was slowly adjusted to 6.0 using diluted phosphoric acid. After cooling down to 55 degrees C, the A. niger derived proline specific endoproteases was added in a concentration of 4 enzyme units/ gram caseinate (see Materials & Methods section for unit definition). After an incubation (with stirring) for 3 hours at 55 degrees C, the pH was lowered to 4.5 by slowly adding concentrated phosphoric acid. In this larger scale preparation the heat treatment step to inactivate the proline specific endoprotease at this part of the process was omitted. Then the suspension was quickly cooled to 4 degrees C and kept overnight (without stirring) at this temperature. The next morning the clear upper layer was decanted and evaporated to reach a level of 40% dry matter. The latter concentrated liquid was subjected to a UHT treatment of 4 seconds at 140 degrees C and then ultrafiltered at 50 degrees C. After germ filtration, the liquid was spray dried. This material is hereinafter referred to as Casein Derived Bio-Active Peptides (CDBAP). Using the LC/MS procedures outlined in the Materials & Methods section, the IPP, LPP and VPP content of the powdered product was determined. According to its nitrogen content, the powdered product has a protein content of about 60 % (using a conversion factor of 6.38). The IPP, LPP and VPP contents of the powder are provided in Table 6. The amino acid composition of the CDBAP product is provided in Table 7. Quite remarkable is the increase of the molar proline content of the spray dried material obtained after acid precipitation: from an initial 12 % to approx 24%.

Table 6: IPP, LPP and VPP content of CDBAP.

| IPP | LPP | VPP |
|---|---|---|
| Tripeptide content in mg / gram powder | | |
| 2.5 | 6.5 | < 0.1 |
| Tripeptide content in mg / gram protein | | |
| 4.2 | 10.8 | < 0.17 |

Table 7: Amino acid composition of the potassium caseinate starting material and CDBAP (amino acid contents after acid hydrolysis and shown as percentages of the molar amino acid content).

| Amino acid | Starting material | CDBAP |
|---|---|---|
| Asp | 6.5 | 3.2 |
| Glu | 18.9 | 12.5 |
| Asn | - | - |
| Ser | 6.7 | 4.3 |
| Gln | - | - |
| Gly | 3.5 | 3.2 |
| His | 2.2 | 3.7 |
| Arg | 2.8 | 2.3 |
| Thr | 4.3 | 3.0 |
| Ala | 4.5 | 3.4 |
| Pro | 12.3 | 24.1 |
| Tyr | 3.9 | 2.4 |
| Val | 7.1 | 9.6 |
| Met | 2.3 | 3.9 |
| Ile | 5.0 | 4.1 |
| Leu | 9.2 | 9.0 |
| Phe | 4.0 | 3.9 |
| Lys | 6.9 | 7.4 |
| Total | 100 | 100 |

The presence of novel ACE inhibiting peptides in CDBAP was investigated by using 2-dimensional-chromatographic-separation combined with an at-line ACE inhibition assay and mass spectrometry for identification. In the first analysis the peptide mixture was separated on an ODS3 liquid chromatography (LC) column and ACE inhibition profiles were generated from the various fractions obtained. In a second analysis the fractions from the first column showing a high ACE inhibition were further separated on a Biosuite LC column using a different gradient profile. The fractions collected from this second column were split into two parts: one part was used for the at-line ACE inhibition measurement while the other part was subjected to MS and MS-MS analysis to identify the peptides present.

All analyses were performed using an Alliance 2795 HPLC system (Waters, Etten-Leur, the Netherlands) equipped with a dual trace UV-detector. For identification of the peptides the HPLC-system was coupled to a Q-TOF mass spectrometer from the same supplier. In the tests 20 $\mu$l of a 10% (w/v) solution of CDBAP in Milli-Q water was injected on a 150 x 2.1 Inertsil 5 ODS3 column with a particle size of 5 $\mu$m (Varian, Middelburg, the Netherlands). Mobile phase A consisted of a 0.1% trifluoroacetic acid (TFA) solution in Milli-Q water. Mobile phase B consisted of a 0.1% TFA solution in acetonitrile. The initial eluent composition was 100% A. The eluent was kept at 100% A for 5 minutes. Then a linear gradient was started in 10 minutes to 5% B, followed by a linear gradient in 10 minutes to 30% B. The column was flushed by raising the concentration of B to 70% in 5 minutes, and was kept at 70% B for another 5 minutes. After this the eluent was changed to 100% A in 1 minute and equilibrated for 9 minutes. The total run time was 50 minutes. The effluent flow was 0.2 ml min$^{-1}$ and the column temperature was set at 60 °C. A UV chromatogram was recorded at 215 nm. Eluent fractions were collected in a 96 well plate using a 1 minute interval time resulting in fraction volumes of 200 $\mu$l. The effluent in the wells was neutralised by addition of 80 $\mu$l of a 0.05% solution of aqueous ammonium hydroxide (25%). The solvent was evaporated until dryness under nitrogen at 50°C. After this the residue was reconstituted in 40 $\mu$l of Milli-Q water and mixed for 1 minute.

For the at-line ACE inhibition assay 27 $\mu$l of a 33.4 mU ml$^{-1}$ ACE (enzyme obtained from Sigma Chemicals) in phosphate buffered saline (PBS) pH 7.4 with a chloride concentration of 260 mM was added and the mixture was allowed to incubate for 5 minutes on a 96 well plate mixer at 700 RPM. After the incubation period 13 $\mu$l of a 0.35 mM hippuric acid-histidine-leucine (HHL) solution in PBS buffer was added and mixed for 1 minute at 700 RPM. The mixture was allowed to react for 60 minutes at 50 °C in a GC-oven. After the reaction the plate was cooled in melting ice.

The 96 well plate was then analysed on a flash-HPLC-column. Of the reaction mixture of each well 30 $\mu$l was injected on a Chromlith Flash RP18e 25 x 4.6 mm HPLC column (Merck, Darmstadt, Germany) equipped with a 10 x4.6 mm RP18e guard column from the same supplier. The isocratic mobile phase consisted of a 0.1 % solution of TFA in water/acetonitrile 79/21. The eluent flow was 2 ml min$^{-1}$ and the column temperature was 25 °C. The injections were performed with an interval time of 1 minute. Hippuric acid (H) and and HHL were monitored at 280 nm. The peak heights of H and HHL were measured and the ACE inhibition (ACEI) of each fraction was calculated according to the equation:

$$ACEI\alpha = \frac{(DC_w - DC_a)}{DC_w * 100}$$

$ACEI\alpha$    Percentage inhibition of the analyte
$Dc_w$    Degree of Cleavage by ACE of HHL to H and HL in water
$Dc_a$    Degree of Cleavage of HHL to H and HL for the analyte

The Degree of Cleavage was calculated by expressing the peak height of H as a fraction of the sum of the peak heights of H and HHL.

The highest ACE inhibition was measured in the fractions eluting between 18 and 26 minutes. This region was collected and re-injected on a 150 x 2.1 mm Biosuite column with a particle size of 3 $\mu$m (Waters, Etten-Leur, the Netherlands). Mobile phase A here consisted of a 0.1% formic acid (FA) solution in Milli-Q water. Mobile phase B consisted of a 0.1% FA solution in methanol. The initial eluent composition was 100% A. The eluent was kept at 100% A for 5 minutes. After this a linear gradient was started in 15 minutes to 5% B, followed by a linear gradient in 30 minutes to 60% B. The eluent was kept at 60% B for another 5 minutes. Finally the eluent was reduced to 100% of mobile phase A in 1 minute and equilibrated for 10 minutes. The total run time was 65 minutes. The eluent flow was 0.2 ml min$^{-1}$ and the column temperature was set at 60 °C. The UV trace was recorded at 215 nm. Fractions were collected from the Biosuite column at 10 seconds interval time. The fractions were again split into two parts, one part was used to measure the activity using the at-line ACE inhibition method described earlier, while the other part was used to identify the active peptides using MS and MS-MS.

Two chromatographic peaks with molecular ions of 326.2080 Da and two other peaks with molecular ions of 330.2029 Da and 318.1488 Da corresponded with the increased ACE inhibition measured in the area between 18 and 26 minutes. Using MS-MS these peptides were identified as the structural isomers IPP and LPP (- 0.6 ppm), ITP (-4.8 ppm) and MAP (+2.8 ppm) respectively. The protein sources of the peptides are kappa-casein f108-110 (IPP), β-casein f151-153 (LPP), α-s2-casein f119-121 (ITP) and β-casein f102-104 (MAP). IPP and LPP were reported earlier as ACE inhibiting peptides with IC50 values of 5 and 9.6 μM respectively (Y. Nakamura, M. Yamamoto., K. Sakai., A. Okubo., S. Yamazaki, T. Takano, J. Dairy Sci. 78 (1995) 777-783; Y. Aryoshi, Trends in Food Science and Technol. 4 (1993) 139-144). However, the tripeptides ITP and MAP were, to our knowledge, never before reported as potent ACE inhibiting peptides.

MAP, ITP and IPP were chemically synthesised and the activity of each peptide was measured using a modified Matsui assay described hereafter

Quantification of MAP and ITP in the various samples was performed on a Micromass Quattro II MS instrument operated in the positive electrospray, multiple reaction monitoring mode. The HPLC method used was similar to the one described above. The MS settings (ESI+) were as follows: cone voltage 37 V, capillary voltage 4 kV, drying gas nitrogen at 300 l/h. Source and nebulizer temperature: 100°C and 250°C, respectively. The synthesized peptides were used to prepare a calibration line using the precursor ion 318.1 and the summed product ions 227.2 and 347.2 for MAP and using the precursor ion 320.2 and the summed product ions 282.2 and 501.2 for ITP. According to these analyses the novel ACE inhibiting tripeptides MAP and ITP are present in the CDBAP product in quantities corresponding with 2.9 mg MAP/gram CDBAP or 4.8 mg MAP/gram protein in CDBAP and 0.9 mg ITP/ gram CDBAP en 1.4 mg ITP/ gram protein in CDBAP.

To determine the ACE inhibition activity of MAP and ITP , the chemically synthesised tripeptides were assayed according to the method of Matsui et al. (Matsui, T. et al. (1992) Biosci. Biotech. Biochem. 56: 517-518) with some minor modifications. The various incubations are shown in Table 8.

Table 8: Procedure for Matsui ACE inhibition assay. The components were added in a 1.5-ml tube with a final volume of 120 μl.

| Component | Control 1 (μl) | Control 2 (μl) | Sample 1 (μl) | Sample 2 (μl) |
|---|---|---|---|---|
| Hip-His-Leu (3 mM) | 75 | 75 | 75 | 75 |
| $H_2O$ | 25 | 45 | - | 20 |
| Inhibiting peptide | - | - | 25 | 25 |
| ACE (0.1 U/ml) | 20 | - | 20 | - |

Each one of the four samples contained 75 μl 3 mM hippuryl histidine leucine (Hip-His-Leu, Sigma Chemicals) dissolved in a 250 mM borate solution containing 200 mM NaCl, pH 8.3. ACE was obtained from Sigma Chemicals. The mixtures were incubated at 37°C and stopped after 30 min by adding 125 μl 0.5 M HCl. Subsequently, 225 μl bicine/ NaOH solution (1 M NaOH : 0.25 M bicine (4:6)) was added, followed by 25 μl 0.1M TNBS (2,4,6-Trinitrobenzenesulfonic acid, Fluka, Switzerland; in 0.1 M $Na_2HPO_4$). After incubation for 20 min. at 37°C, 4 ml 4 mM $Na_2SO_3$ in 0.2 M $NaH_2PO_4$ was added and the light absorbance at 416 nm was measured with UV/Vis spectrophotometer (Shimadzu UV-1601 with a CPS controller, Netherlands).

The amount of ACE inhibition (ACEI) activity was calculated as a percentage of inhibition compared with the conversion rate of ACE in the absence of an inhibitor according to the following formula:

$$\text{ACEI (\%)} = ((\text{Control1-Control 2})-(\text{Sample 1-Sample 2}))/(\text{Control 1-Control 2}) * 100$$

wherein

Control 1 = Absorbance without ACE inhibitory component (= max. ACE activity) [AU].

Control 2 = Absorbance without ACE inhibitory component and without ACE (background) [AU].

Sample 1 = Absorbance in the presence of ACE and the ACE inhibitory component [AU].

Sample 2 = Absorbance in the presence of the ACE inhibitory component, but without ACE [AU].

The $IC_{50}$ of the chemically synthesized MAP and ITP tripeptides as obtained are shown in Table 9 together with IC50 values obtained in the at-line measurements used in the screening phase of the experiment. The measurement of chemically synthesized IPP was included as an internal reference for the various measurements.

Table 9: ACE inhibition (IC50 values) of MAP, ITP and IPP values determined by the at-line ACE assay and the modified Matsui assay.

| Peptide | IC50 value in $\mu$M | |
|---|---|---|
| | at-line ACE assay | Modified Matsui assay |
| MAP | 3.8 | 0.4 |
| ITP | 50 | 10 |
| IPP (reference) | 7.1 | 2 |

Example 8

Novel ACE inhibiting peptides MAP and ITP are likely to survive in the human gastrointestinal tract

After consumption, dietary proteins and peptides are exposed to various digestive enzymatic processes in the gastrointestinal tract. In order to assess the stability of the newly identified bioactive peptides in the human gastrointestinal tract, the CDBAP preparation (prepared as described in Example 7) was subjected to a gastro-intestinal treatment (GIT) simulating the digestive conditions typically found in the human body. Samples obtained after various incubation times in the GIT model system were analysed using the on-line HPLC-Bioassay-MS or HRS-MS system to quantify any residual MAP and ITP peptides. The GIT procedure was performed in a standardized mixing device incorporating a 100ml flask (as supplied by Vankel, US). The temperature of the water bath was set to 37.5°C and the paddle speed was chosen such that the sample was kept in suspension (100 rpm).
About 3.4 grams of CDBAP (protein level of approx 60 %) was dissolved / suspended in 100 ml Milli-Q water. During gastric simulation 5 M HCl was used to decrease the pH. At the end of gastric simulation and during the duodenal phase 5 M NaOH was used to raise the pH.
The CDBAP suspension was preheated to 37.5°C and 5 ml of the suspension was removed to dissolve 0.31 g of pepsin (Fluka order no. 77161). At t= 0 min the 5 ml with the now dissolved pepsin was added back to the suspension.Then the pH of the CDBAP suspension was adjusted slowly by hand using a separate pH meter according to the following scheme:

t = 20 min    pH decreased to 3.5
t = 40 min    pH to 3.0
t = 50 min    pH to 2.3
t = 60 min    pH to 1.8
t = 65 min    pH raised to 2.7
t = 75 min    pH to 3.7
t = 80 min    pH to 5.3

At t = 90 min 0.139 g of 8 times USP pancreatin (Sigma order no. P7545) was carefully mixed in another 5 ml of the CDBAP suspension and immediately added back.. The incubation continued according to the following scheme:

t = 93 min    pH to 5.5
t = 95 min    pH to 6.3
t = 100 min    pH to 7.1

The experiment was stopped at t = 125 min and the pH was checked (was still pH 7).
Then the samples were transferred into a beaker and were placed in a microwave till boiling. Subsequently, the samples were transferred into glass tubes and incubated at 95°C for 60 min to inactivate all protease activity. After cooling the samples were put in Falcon tubes and centrifuged for 10 min at 3000 x g. The supernatant was freeze dried. The total N concentration of the powder as obtained was determined and converted to protein level using the Kjeldahl factor of casein (6.38). According to these data the protein level of the CDBAP preparation after the GIT procedure was 48.4%. The levels of MAP and ITP surviving the proteolytic treatment according to the GIT procedure were determined as decribed in Example 7 and the data obtained are shown in Table 10.

According to the results of the experiment both MAP and ITP exhibit a high resistance against GIT digestion. In combination with the low IC50 values for these tripeptides (also as determined in Example 7), the data suggest considerable potential for the two novel ACE inhibiting peptides as blood pressure lowering peptides.

Table 10: Concentrations of MAP and ITP before and after passage through a simulated human gastro-intestinal tract (GIT procedure)

| Sample | Concentration in $\mu$g g$^{-1}$ powder | |
|---|---|---|
| | MAP | ITP |
| | | |
| CDBAP (Example 7) | 2851.4 | 903.7 |
| CDBAP after GIT | 3095.8 | 889.1 |

**Claims**

1. The tripeptide ITP and/or a salt of MAP and/or a salt of ITP.

2. A protein hydrolysate comprising MAP and/or ITP or a salt of MAP and/or a salt of ITP which has a degree of hydrolysis (DH) of 5 to 50%, preferably 10 to 40%, more preferably a DH of 20 to 35%.

3. A peptide mixture comprising MAP and/or ITP or a salt of MAP and/or a salt of ITP and which comprises
   at least 1 mg MAP/gram protein, preferably
   at least 2 mg MAP/gram protein, more preferably
   at least 4 mg MAP/gram protein.

4. A peptide mixture according to claim 3 whereby the amount of peptides having a MW of less than 500Da is at least 30 wt% (dry matter) of the peptide mixture, preferably between 35 and 70%wt (dry matter) of the peptide mixture.

5. A peptide mixture according to claim 3 or 4 or a protein hydrolysate according to claim 2 which further comprises IPP or LPP.

6. A peptide mixture according to any one of claims 3 to 5 or a protein hydrolysate according to claim 2 which comprises 1 to 90% of water, preferably 1 to 30% of water, more preferably 1 to 15% of water.

7. A method to produce the tripeptide MAP and/or ITP and/or a salt thereof, which comprises the enzymatic hydrolysis of a protein and optionally converting MAP and/or ITP into its salt which comprises the use of a proline specific protease to hydrolyze a suitable protein.

**Patentansprüche**

1. Tripeptid ITP und/oder Salz von MAP und/oder Salz von ITP.

2. Proteinhydrolysat, umfassend MAP und/oder ITP oder ein Salz von MAP und/oder ein Salz von ITP, das einen DH-Wert (Hydrolysegrad) von 5 bis 50%, vorzugsweise 10 bis 40%, stärker bevorzugt einen DH-Wert von 20 bis 35% aufweist.

3. Peptidgemisch, umfassend MAP und/oder ITP oder ein Salz von MAP und/oder ein Salz von ITP, das Folgendes umfasst:

   mindestens 1 mg MAP/Gramm Protein, vorzugsweise
   mindestens 2 mg MAP/Gramm Protein, stärker bevorzugt
   mindestens 4 mg MAP/Gramm Protein.

4. Peptidgemisch nach Anspruch 3, wobei die Menge an Peptiden mit einem MW-Wert von weniger als 500 Da mindestens 30 Gew.-% (Trockenmasse) des Peptidgemischs, vorzugsweise zwischen 35 und 70 Gew.-% (Trockenmasse) des Peptidgemischs beträgt.

5. Peptidgemisch nach Anspruch 3 oder 4 oder Proteinhydrolysat nach Anspruch 2, das ferner IPP oder LPP umfasst.

6. Peptidgemisch nach einem der Ansprüche 3 bis 5 oder Proteinhydrolysat nach Anspruch 2, das 1 bis 90% Wasser, vorzugsweise 1 bis 30% Wasser, stärker bevorzugt 1 bis 15% Wasser umfasst.

7. Verfahren zur Herstellung des Tripeptids MAP und/oder ITP und/oder eines Salzes davon, das die enzymatische Hydrolyse eines Proteins und gegebenenfalls die Umwandlung von MAP und/oder ITP in sein Salz umfasst, wobei eine prolinspezifische Protease zur Hydrolyse eines geeigneten Proteins verwendet wird.

**Revendications**

1. Tripeptide ITP et/ou un sel de MAP et/ou un sel de ITP.

2. Hydrolysat de protéines comprenant MAP et/ou ITP ou un sel de MAP et/ou un sel de ITP qui a un degré d'hydrolyse (DH) de 5 à 50 %, de préférence de 10 à 40 %, plus préférablement un DH de 20 à 35 %.

3. Mélange de peptides comprenant MAP et/ou ITP ou un sel de MAP et/ou un sel de ITP et qui comprend
   au moins 1 mg de MAP/gramme de protéine, de préférence
   au moins 2 mg de MAP/gramme de protéine, plus préférablement
   au moins 4 mg de MAP/gramme de protéine.

4. Mélange de peptides selon la revendication 3, **caractérisé en ce que** la quantité de peptides ayant un PM inférieur à 500Da est au moins 30 % en poids (matière sèche) du mélange de peptides, de préférence entre 35 et 70 % en poids (matière sèche) du mélange de peptides.

5. Mélange de peptides selon la revendication 3 ou 4 ou un hydrolysat de protéines selon la revendication 2 qui comprend en outre IPP ou LPP.

6. Mélange de peptides selon l'une quelconque des revendications 3 à 5 ou un hydrolysat de protéines selon la revendication 2 qui comprend de 1 à 90 % d'eau, de préférence de 1 à 30 % d'eau, plus préférablement de 1 à 15 % d'eau.

7. Méthode de production du tripeptide MAP et/ou ITP et/ou un sel de ceux-ci, qui comprend l'hydrolyse enzymatique d'une protéine et éventuellement la transformation de MAP et/ou de ITP en son sel qui comprend l'utilisation d'une protéase spécifique de la proline pour hydrolyser une protéine convenable.

Fig. 1

pH optimum proline specific endoprotease
of *A.niger* at 37 °C
Z-Gly-Pro-pNA

Fig.2

Specificity profile of EndoPro tested at pH 4, on AAX-pNA substrates, where X is all natural amino acids (1:300 dilution) also including AP-pNA and Z-G-P-pNA

## Fig.3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 02036127 A
- EP 0583074 A
- WO 0245523 A

- WO 0245524 A

### Non-patent literature cited in the description

- **Ondetti, M.A. et al.** *Science,* 1977, vol. 196, 441-444
- **Maruyama, S. ; Suzuki, H.** *Agric Biol Chem.,* 1982, vol. 46 (5), 1393-1394
- *J. Dairy Sci.,* 1995, vol. 78, 777-783
- **Nakamura et al.** *J. Dairy Sci.,* 1995, vol. 78, 12531257
- Peptides: Chemistry and Biology. Wiley-VCH Verlag GmbH, 2002
- **Y. Poilot et al.** *Journal of Membrane Science,* 1999, vol. 158, 105-114
- **Fulop et al.** *Cell,* 24 July 1998, vol. 94, 161-170
- **Sambrook et al.** Molecular Cloning: A Laboratory Manual. Cold Spring Harbor Laboratory Press, 1989

- **Rawling,N.D. ; Barrett, A.J.** *Biochim. Biophys. Acta,* 1996, vol. 1298, 1-3
- Pharmaceutical Dosage Forms. Marcel Dekker, Inc, vol. 1, 2, 3
- *World Journal of Microbiology & Biotechnology,* 1995, vol. 11, 209-212
- **Nielsen et al.** *JFS,* 2001, vol. 66 (5), 642-646
- **Y. Nakamura ; M. Yamamoto ; K. Sakai ; A. Okubo ; S. Yamazaki ; T. Takano.** *J. Dairy Sci.,* 1995, vol. 78, 777-783
- **Y. Aryoshi.** *Trends in Food Science and Technol.,* 1993, vol. 4, 139-144
- **Matsui ; Matsui, T. et al.** *Biosci. Biotech. Biochem.,* 1992, vol. 56, 517-518